# EUROPÄISCHE PATENTSCHRIFT

(11) **EP 2 627 362 B1**
(45) Veröffentlichungstag und Bekanntmachung des Hinweises auf die Patenterteilung: **10.12.2014**
(21) Anmeldenummer: 11784435.7
(22) Anmeldetag: 17.10.2011
(51) Int. Cl.: A61L 12/08, A61L 12/12, B65D 51/16, B65D 51/18

(54) **BEHÄLTNIS FÜR EINE KONTAKTLINSEN-AUFBEWAHRUNGSFLÜSSIGKEIT UND REINIGUNGS- UND/ODER ENTKEIMUNGSVORRICHTUNG FÜR IN EINER KONTAKTLINSENAUFBEWAHRUNGSFLÜSSIGKEIT BEFINDLICHE KONTAKTLINSEN**
CONTAINER FOR A CONTACT LENS STORAGE SOLUTION AND CLEANING AND/OR STERILIZING DEVICE FOR CONTACT LENSES IN A CONTACT LENS STORAGE SOLUTION
CONTENANT POUR UN LIQUIDE DE CONSERVATION DE LENTILLES DE CONTACT ET DISPOSITIF DE NETTOYAGE ET/OU DE DÉCONTAMINATION POUR DES LENTILLES DE CONTACT SE TROUVANT DANS UN LIQUIDE DE CONSERVATION DE LENTILLES DE CONTACT

(30) Priorität: 27.01.2011 EP 11152402; 15.10.2010 EP 10187698
(43) Veröffentlichungstag der Anmeldung: 21.08.2013
(73) Patentinhaber: Sturm, Albert, 50931 Köln (DE); Nold, Yves, 75017 Paris (FR)
(72) Erfinder: Sturm, Albert, 50931 Köln (DE); Nold, Yves, 75017 Paris (FR)
(74) Vertreter: Von Kreisler Selting Werner - Partnerschaft von Patentanwälten und Rechtsanwälten mbB
(86) Internationale Anmeldenummer: PCT/EP2011/068074
(87) Internationale Veröffentlichungsnummer: WO 2012/049323

(56) Entgegenhaltungen:
- CA-A1- 2 120 628
- DE-U1-202009 003 699
- US-A- 3 473 886
- US-A- 4 637 919
- US-A1- 2007 215 652

## Beschreibung

Die Erfindung betrifft ein Behältnis für eine Kontaktlinsen-Aufbewahrungsflüssigkeit, insbesondere mit in dieser befindlichen Kontaktlinsen. Ferner betrifft die Erfindung eine Reinigungs- und/oder Entkeimungsvorrichtung für in einer Kontaktlinsenaufbewahrungsflüssigkeit befindliche Kontaktlinsen.

Die Reinigung von insbesondere weichen Kontaktlinsen wird durch verschiedene Systeme gewährleistet. Weit verbreitet sind dabei wässrige Systeme, bei denen ein gelöstes oxidatives Reagenz auf die Kontaktlinse einwirkt und danach entfernt werden muss. Üblicherweise wird hierbei als oxidatives Reagenz aktiver Sauerstoff beispielsweise in Form von Wasserstoffperoxid oder Ozon eingesetzt. Im ersten Fall muss das überschüssige Wasserstoffperoxid durch die Zugabe anderer Reagenzien zerstört werden, um nicht versehentlich mit Wasserstoffperoxid kontaminierte Kontaktlinsen dem Anwender zuzuführen. Als Wasserstoffperoxid zerstörende Agentien werden Reduktionsmittel wie Natriumthiosulfat eingesetzt, die ihrerseits wiederum ausgespült werden müssen. Praktischer ist hier in der Anwendung der Einsatz von Ozon, da dann die umständliche Zerstörung des Wasserstoffperoxids unterbleiben kann.

US4637919 offenbart ein verschließbares Behältnis für eine Kontaktlinsen-Aufbewahrungsflüssigkeit und zur Kontaktlinsendesinfektion umfassend einen Behälter mit Öffnung im Deckel und Kontaktlinsen-Halter.

EP 968 003 B1 offenbart eine effiziente Reinigung von Kontaktlinsen durch den simultanen Einsatz von Proteasen und Wasserstoffperoxid. Dabei zerstören Proteasen hartnäckige Proteinablagerungen in den Kontaktlinsen und das Wasserstoffperoxid desinfiziert die danach gereinigte Kontaktlinse. Nachteilig daran ist auch wiederum die Verwendung von Wasserstoffperoxid, das neutralisiert werden muss.

Weiterhin nachteilig an der Verwendung des Wasserstoffperoxids ist dessen Labilität, da es sich bei Lagerung langsam in Wasser und Sauerstoff zersetzt. Dies ist insbesondere in Ländern mit hohen Durchschnittstemperaturen zu beachten.

Wünschenswert ist es, wenn die oben beschriebene Reinigung von Kontaktlinsen in einem Behältnis erfolgen könnte, in dem sich die zu reinigenden Kontaktlinsen in einer Aufbewahrungsflüssigkeit befinden. Ein solches Behältnis könnte von der die Kontaktlinsen benötigenden Person mitgeführt werden, um dann zum Zwecke der Reinigung der Kontaktlinsen mit einem Wasserstoffperoxyd- oder Ozongenerator verbunden zu werden. Derartige Generatoren sind grundsätzlich bekannt und zum Teil im Handel erhältlich.

Aus DE 20 2009 003 699 U1 ist ein Behältnis für eine Kontaktlinsen-Aufbewahrungsflüssigkeit, insbesondere mit in dieser befindlichen Kontaktlinsen bekannt, bei dem über sich selbstständig öffnende bzw. schließende Ventile eine Begasung mit einem Reinigungs- und/oder Entkeimungsgas erfolgen kann, wenn das Behältnis in den Aufnahmeraum eines Generators zur Erzeugung des Reinigungs- und/oder Entkeimungsgases gesetzt wird.

Aufsetzstopfen mit verschließbaren Öffnungen für Behälter sind z. B. aus USA-2007/0215652 bekannt.

Aufgabe der Erfindung ist es, ein Behältnis für eine Kontaktlinsen-Aufbewahrungsflüssigkeit zu schaffen, das sich auf bequeme Art und Weise für die Zufuhr eines Reinigungs-/Entkeimungsgases eignet.

Zur Lösung dieser Aufgabe wird mit der Erfindung ein Behältnis für eine Kontaktlinsen-Aufbewahrungsflüssigkeit vorgeschlagen, das versehen ist mit den Merkmalen des Anspruchs 1. Einzelne Ausgestaltungen der Erfindung sind Gegenstand der Unteransprüche.

Erfindungsgemäß weist das Behältnis ein "Doppelverschluss-System" auf, das ein erstes und ein zweites Verschlusselement umfasst. Das erste Verschlusselement dient zum Verschließen der Befüllöffnung des Gefäßes und weist einen Gaszuführkanal auf, der sich zwischen einem Einlass und einem Auslass durch das Verschlusselement hindurch erstreckt. Sowohl der Einlass als auch der Auslass sind mit Hilfe des zweiten Verschlusselements wahlweise verschließbar.

Ferner ist nach der Erfindung vorgesehen, dass an dem ersten Verschlusselement ein Kontaktlinsenhalter angeordnet ist, durch den sich ein Gasweiterleitungskanal erstreckt, welcher in Fluidverbindung mit dem Gaszuführkanal des ersten Verschlusselements steht. Der Kontaktlinsenhalter ist zweckmäßigerweise mit zwei Haltebereichen für Kontaktlinsen versehen, die in Form von Klappen oder dergleichen ausgebildet sind. Die Klappen weisen eine Gitterstruktur auf und sind von der Aufbewahrungsflüssigkeit umspült. Der Gasweiterleitungskanal innerhalb des Kontaktlinsenhalters erstreckt sich zweckmäßigerweise bis zu einem unterhalb der Aufbewahrungsklappen befindlichen Auslass, so dass das aus diesem Auslass aufsteigende Gas an den Kontaktlinsen vorbeiströmt, um oben aus dem Auslass des ersten Verschlusselements auszutreten.

Die Bedienung des erfindungsgemäßen Behältnisses ist denkbar einfach. Zum Transport des Behältnisses mit in diesem befindlicher Aufbewahrungsflüssigkeit sowie befindlichen Kontaktlinsen ist die Befüllöffnung mit Hilfe des ersten Verschlusselements dicht verschlossen, während das zweite Verschlusselement den Einlass und den Auslass des Gaszuführkanals des ersten Verschluss-elements verschließt. Soll nun ein Reinigungs- bzw. Entkeimungsgas in das Gefäß eingeleitet werden, so bedarf es lediglich des Freilegens bzw. Öffnens des Einlasses des Gaszuführkanals des ersten Verschlusselements sowie der Freigabe des Auslasses durch Überführung des zweiten Verschlusselements aus der Verschlussposition in die Öffnungsposition. Zum Befüllen des Gefäßes mit Aufbewahrungsflüssigkeit bzw. zum Entnehmen der Kontaktlinsen oder zum Einbringen der Kontaktlinsen wird das erste Verschlusselement aus seiner Verschlussposition, in der es die Befüllöffnung verschließt, in seiner Öffnungs-bzw. Freigabeposition überführt, in der die Befüllöffnung freiliegt.

Zum Begasen des Gefäßes wird dieses in eine Reinigungs- und/oder Entkeimungsvorrichtung eingesetzt, die eine Aufnahmekammer für das Gefäß aufweist. Der freigelegte Einlass des Gaszuführkanals des ersten Verschlusselements befindet sich in der Aufnahmekammer, die im Übrigen gegenüber der Umgebung abgedichtet ist, wenn sich das Gefäß in der Aufnahmekammer befindet. Das über den Einlass des Gaszuführkanals des ersten Verschlusselements eindringende Gas gelangt über den Auslass des ersten Verschlusselements wieder aus dem Gefäß heraus. Insoweit praktisch und einfach handhabbar ist daher das erfindungsgemäße Behältnis dadurch, dass sowohl der Einlass als auch der Auslass des Gaszuführkanals durch Überführen des zweiten Verschlusselements in die Öffnungsposition freigelegt und durch Überführen in die Verschlussposition verschlossen werden kann.

In vorteilhafter Weiterbildung der Erfindung ist ferner vorgesehen, dass die Außenseite des ersten Verschlusselements in dessen an dem Gefäß angebrachten Zustand einen von dem Gefäß aufragenden ersten Außenflächenbereich aufweist, innerhalb dessen der mindestens eine Einlass angeordnet ist. Hierbei ist es insbesondere zweckmäßig, dass die Außenseite des ersten Verschlusselements in dessen an dem Gefäß angebrachten Zustand einen im Wesentlichen parallel zur Erstreckung der Befüllöffnung verlaufenden zweiten Außenflächenbereich aufweist, innerhalb dessen die Entlüftungsöffnung des ersten Verschlusselements angeordnet ist. Das erste Verschlusselement ist zweckmäßigerweise als Kappe ausgebildet, die eine Umfangsseite und eine Stirnseite aufweist. Die Umfangsseite bildet den zuvor genannten ersten Außenflächenbereich, während die Stirnseite den zuvor genannten zweiten Außenflächenbereich bildet.

In vorteilhafter Weiterbildung ist des Weiteren vorgesehen, dass das zweite Verschlusselement ebenfalls nach Art einer Kappe ausgebildet ist, die auf das erste Verschlusselement aufgesetzt ist. Im vollständig aufgesetzten Zustand überdeckt dann das zweite Verschlusselement sowohl den Gaszuführkanal-Einlass an der Umfangswand des ersten Verschlusselements als auch den Auslass an der Stirnseite des ersten Verschlusselements.

Bei einer weiteren vorteilhaften Ausgestaltung der Erfindung ist vorgesehen, dass das Gefäß eine Bodenwand mit einer von dieser aufragenden umlaufenden Mantelwand, insbesondere Zylindermantelwand, aufweist, und dass die Befüllöffnung des Gefäßes der Bodenwand gegenüberliegend angeordnet ist. Hierbei ist es ferner von Vorteil, wenn die Mantelwand des Gefäßes einen dessen Bodenwand gegenüberliegenden umlaufenden Rand aufweist, der die Befüllöffnung begrenzt.

Insbesondere zweckmäßig ist es, wenn beide Verschlusselemente jeweils als Schraubverschlussdeckel und insbesondere als Schraubkappen ausgebildet sind. Alternativ kann vorgesehen sein, dass das erste Verschlusselement auf das Gefäß aufsteck- oder aufsetzbar ist oder in die Befüllöffnung einsteckbar oder an dem Gefäß durch Verdrehen, beispielsweise mittels einer Bajonett-Verbindung, anbringbar ist und/oder dass das zweite Verschlusselement auf das erste Verschlusselement aufsteck- oder aufsetzbar verschraubbar oder an dem ersten Verschlusselement durch Verdrehen, beispielsweise mittels einer Bajonett-Verbindung, anbringbar ist.

Zum Verschließen des Einlasses sowie des Auslasses des Gaszuführkanals des ersten Verschlusselements können Dichtelemente am ersten Verschlusselement und/oder am zweiten Verschlusselement eingesetzt werden. Insbesondere kann vorgesehen sein, dass der Einlass des Gaszuführkanals des ersten Verschlusselements als zumindest teilweise um das Verschlusselement umlaufende Ringnut in dem ersten Außenflächenbereich ausgebildet ist und dass das erste Verschlusselement ein Dichtelement aufweist, an dem das zweite Verschlusselement beim Überdecken der Ringnut dichtend anliegt.

Bei einer weiteren vorteilhaften Ausgestaltung der Erfindung ist vorgesehen, dass das zweite Verschlusselement eine Auslassöffnung aufweist, die seitlich versetzt zur Entlüftungsöffnung des ersten Verschlusselements angeordnet ist, und dass um die Entlüftungsöffnung des ersten Verschlusselements und/oder um die Auslassöffnung des zweiten Verschlusselements herum oder an dem zweiten Verschlusselement der Entlüftungsöffnung gegenüberliegend und/oder an dem ersten Verschlusselement der Auslassöffnung gegenüberliegend ein Dichtungselement angeordnet ist, an dem das zweite bzw. das ersten Verschlusselement in der Verschlussposition des zweiten Verschlusselements dichtend anliegt.

Bei Ausbildung der beiden Verschlusselemente jeweils als Schraubkappen ist es von Vorteil, wenn der Einlass des Gaszuführkanals der ersten Schraubkappe an deren Umfangsabschnitt ausgebildet ist und der Umfangsabschnitt der zweiten Schraubkappe den Einlass des Gaszuführkanals der ersten Schraubkappe zum Verschließen des Einlasses überdeckt und wenn die Entlüftungsöffnung der ersten Schraubkappe in deren Stirnseitenabschnitt ausgebildet ist und der Stirnseitenabschnitt der zweiten Schraubkappe die Entlüftungsöffnung der ersten Schraubkappe zum Verschließen der Entlüftungsöffnung insbesondere mittels eines Dichtungselements überdeckt.

Wie bereits oben erwähnt, ist es von der Bedienung her recht einfach, wenn jedes Verschlusselement manuell aus einer Öffnungsposition in eine Verschlussposition und umgekehrt überführbar ist, wobei beide Verschlusselemente in der jeweiligen Verschlussposition an dem Gefäß bzw. dem ersten Verschlusselement verbleiben und ein mechanischer Widerstand gegen eine aus der betreffenden Öffnungsposition kommende Überführung über die betreffende Verschlussposition hinaus gegeben ist.

Wie ebenfalls bereits oben angedeutet, wird das erfindungsgemäße Behältnis zum Begasen in die Aufnahmekammer einer Einheit zum Erzeugen und/oder Freigeben eines Reinigungs- und/oder Entkeimungsgases eingesetzt. Die Aufnahmekammer weist zu diesem Zweck eine Einsetzöffnung auf, wobei das zweite Verschlusselement ein Anlageelement zur Anlage an der Einsetzöffnung der Aufnahmekammer bei von dieser unter Bildung eines Ringraums zwischen dem Einlass des Gaszuführkanals des ersten Verschlusselements und der Aufnahmekammer aufgenommenen Gefäß aufweist und wobei zwischen der Einsetzöffnung und dem Einlass des Gaszuführkanals des ersten Verschlusselements ein den Ringraum zur Einsetzöffnung hin abdichtendes Dichtungselement angeordnet ist. Das Dichtungselement kann in der Einsetzöffnung der Aufnahmekammer oder um die Einsetzöffnung der Aufnahmekammer herum zur Anlage an dem Anlageelement des zweiten Verschlusselements oder an dem ersten Verschlusselement zwischen der mindestens einen Einlassöffnung des Gaszuführkanals und dem Anlageelement des zweiten Verschlusselements angeordnet sein.

Die Erfindung wird nachfolgend anhand zweier Ausführungsbeispiele und unter Bezugnahme auf die Zeichnung näher erläutert. Im Einzelnen zeigen dabei:
- Fig. 1: eine Seitenansicht einer Einheit zur Erzeugung eines Reinigungs- und/oder Entkeimungsgases wie beispielsweise Wasserstoffperoxid oder Ozon mit einer aufgesetzten Aufnahmekammer für ein Behältnis gemäß einem Ausführungsbeispiel der Erfindung,
- Fig. 2: einen Längsschnitt entlang der Linie II-I des Behältnisses gemäß Fig. 1,
- Fig. 3: einen gegenüber Fig. 2 um 90° versetzt verlaufenden Längsschnitt durch das Behältnis gemäß Fig. 1,
- Fig. 4: eine Schnittansicht entlang der Linie IV-IV der Fig. 3,
- Fig. 5: eine Seitenansicht des Behältnisses bei in seiner Verschlussposition befindlichem zweiten Verschlusselement,
- Fig. 6: einen Längsschnitt durch das sich im verschlossenen Zustand befindlichen Behältnis entlang der Linie VI-VI der Fig. 5,
- Fig. 7: einen gegenüber Fig. 6 um 90° versetzten Längsschnitt durch das sich im verschlossenen Zustand befindlichen Behältnis,
- Fig. 8: das Behältnis, wie es in den vorherigen Figuren dargestellt ist, wobei der Doppelverschluss abgenommen und das Behältnis somit offen ist und
- Fig. 9: eine alternativ ausgeführt Einheit zur Erzeugung eines Reinigungs- und/oder Entkeimungsgases mit einstückig an diesem ausgebildeter Aufnahmekammer für das Behältnis.

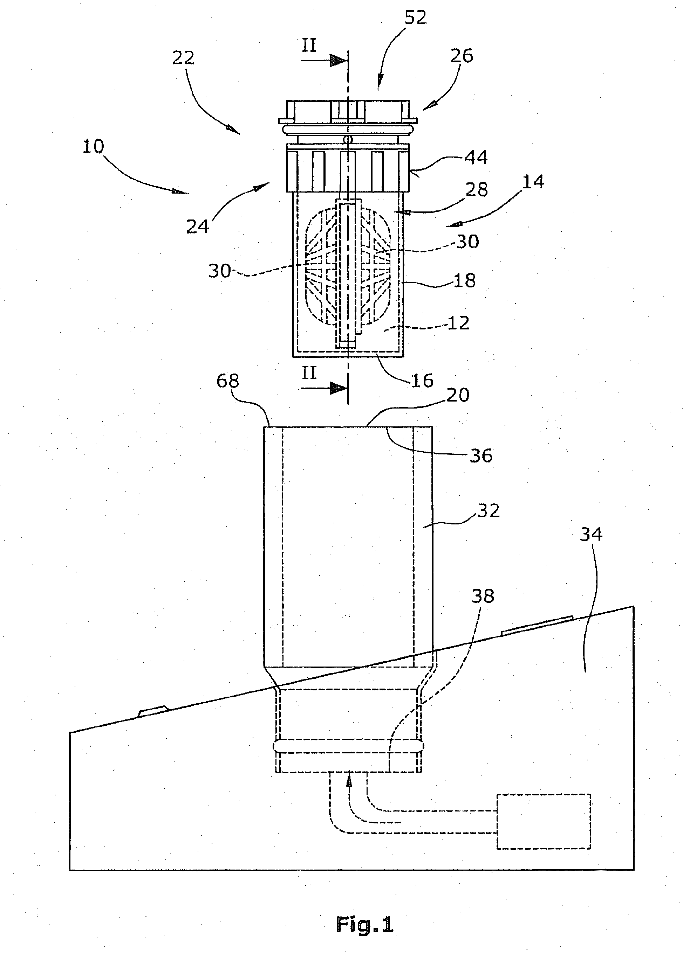

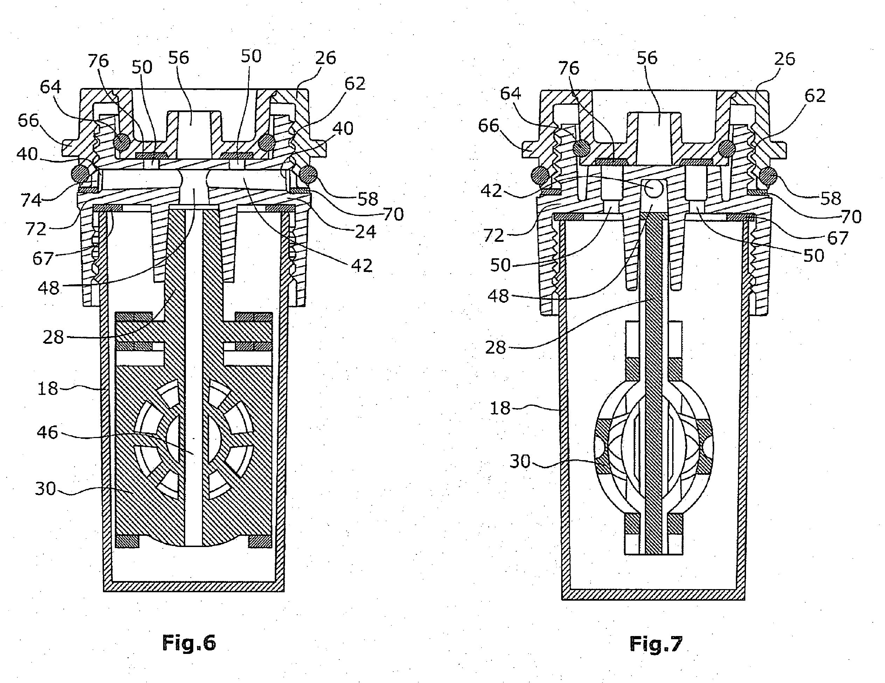

In den Fign. 1 bis 4 sind verschiedene Darstellungen eines Behältnisses 10 für eine Kontaktlinsen-Aufbewahrungsflüssigkeit 12 wiedergegeben, bei der es sich beispielsweise um eine physiologische Kochsalzlösung handelt. Das in diesem Ausführungsbeispiel röhrenförmige Behältnis 10 umfasst ein in diesem Fall im Wesentlichen zylindrisches Gefäß 14 mit einer Bodenwand 16 und einer zylindrischen Mantelwand 18, die von der Bodenwand 16 aufragt. Das Gefäß 14 weist an seinem der Bodenwand 16 gegenüberliegenden Ende eine Befüllöffnung 20 auf (siehe beispielsweise auch Fig. 7), über die das Gefäß 14 mit der Aufbewahrungsflüssigkeit gefüllt werden kann. Eine Doppelverschlussanordnung 22, die weiter unten noch näher beschrieben werden wird, lässt sich durch in diesem Ausführungsbeispiel Verschraubung mit dem Gefäß 14 dicht verbinden, um die Befüllöffnung 20 zu verschließen. Die Doppelverschlussanordnung 22 weist zwei jeweils im Wesentlichen schraubkappenförmig ausgebildete Verschlusselemente 24 und 26 auf. Vom ersten Verschlusselement 24 aus erstreckt sich ein Kontaktlinsenhalter 28, der zur Unterbringung zweier (nicht dargestellter) Kontaktlinsen zwei gitterförmige Halbschalen oder Kappen 30 aufweist, die schwenkbar angeordnet sind und jeweils eine Kontaktlinse umgeben und auf diese Weise halten.

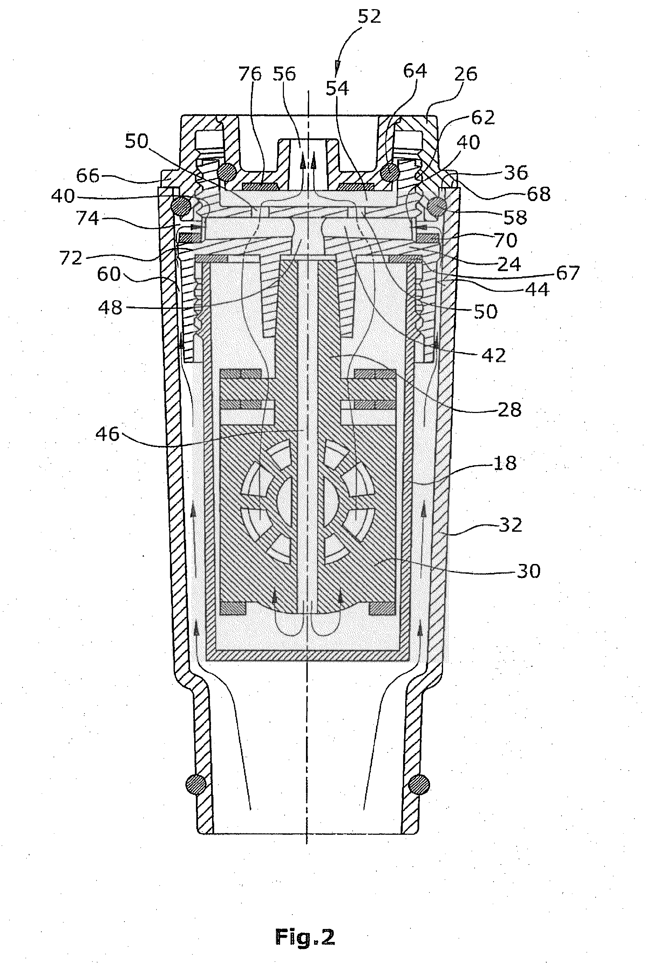

Die Konstruktion und die Funktionsweise zum Begasen des Behältnisses 10 sowie zum Verschließen des Behältnisses 10 ist am besten anhand der Fign. 2 bis 6 zu erkennen. Zum Zwecke der Begasung wird das Behältnis 10 in eine (Aufsetz-)Aufnahmekammer 32 einer Einheit 34 zur Erzeugung des Reinigungs- und/oder Entkeimungsgases eingesetzt. Die Aufnahmekammer 32 weist eine Einsetzöffnung 36 auf. In die Kammer 32 wird über deren Boden 38 das Gas eingeleitet.

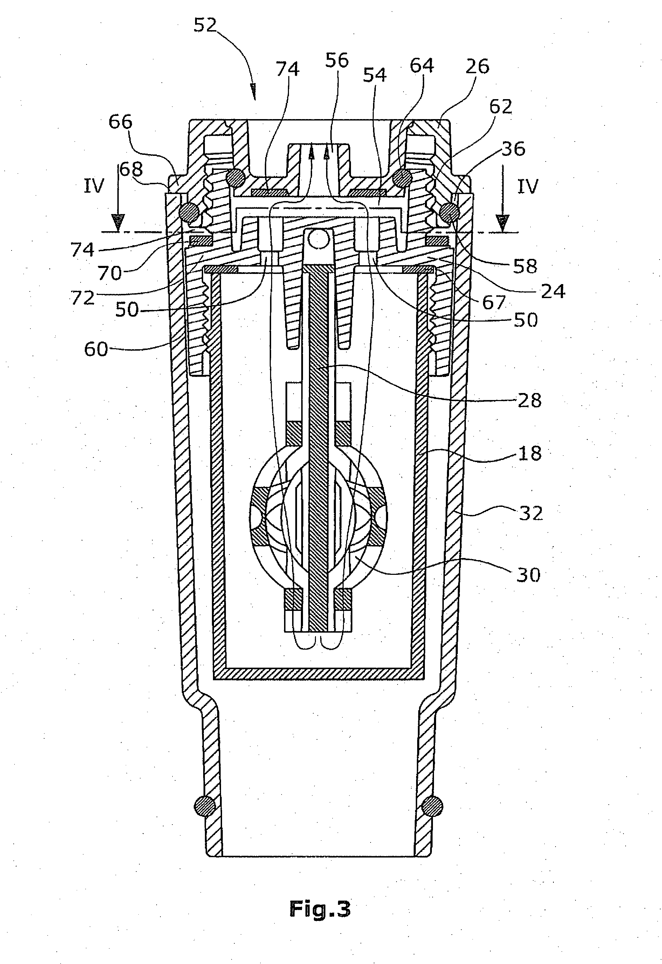

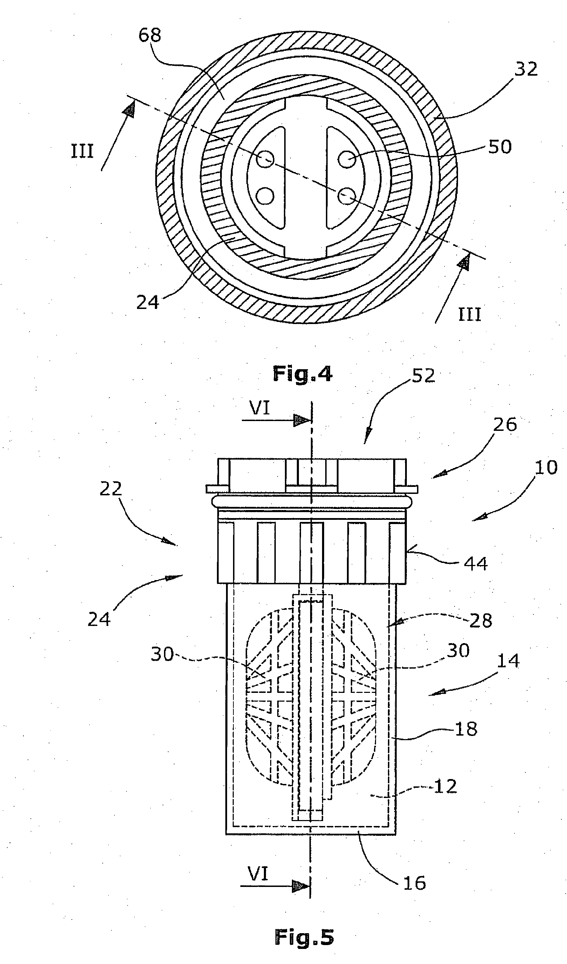

Damit nun das in die Aufnahmekammer 32 eingeleitete Gas in das Behältnis 10 gelangt, weist dieses einen Einlass für das Gas auf. Dieser Einlass muss freigelegt sein, was durch Überführung des zweiten Verschlusselements 26 aus der Verschlussposition gemäß den Fign. 5, 6 und 7 in die Öffnungsposition gemäß den Fign. 1, 2 und 3 erfolgt. In der Öffnungsposition des zweiten Verschlusselements 26 legt dieses in diesem Ausführungsbeispiel zwei Einlässe 40 eines Gaszuführkanals 42 des ersten Verschlusselements 24 frei. Dabei sind die Einlässe 40 im Umfangsbereich 44 (erster Außenflächenbereich) des ersten Verschlusselements 24 ausgebildet. Der Gaszuführkanal 42 erstreckt sich im Wesentlichen diametral durch das erste Verschlusselement 24 hindurch und steht in Fluidverbindung mit einem Gasweiterleitungskanal 46 durch den Kontaktlinsenhalter 28 (siehe beispielsweise die Fig. 2). Der unterhalb der Halbschalen bzw. Kappen 30 endende Gasweiterleitungskanal 46 ist am Gasauslass 48 des Gaszuführkanals 42 mit diesem fluidisch verbunden. Das aus dem Gasweiterleitungskanal 46 austretende Gas steigt innerhalb des Gefäßes 14 auf und dabei an den zu reinigenden Kontaktlinsen entlang. Es entweicht über Entlüftungsöffnungen 50 im ersten Verschlusselement 24, die beidseitig des Gaszuführkanals 42 angeordnet sind und in der Stirnseite 52 (zweiter Außenflächenbereich) des ersten Verschlusselements 24 enden (siehe Fign. 3 und 4). Im geöffneten Zustand der Einlässe 40 gibt das zweite Verschlusselement 26 auch die Entlüftungsöffnungen 50 frei, so dass das Gas entweichen kann. Das Gas entweicht durch die Entlüftungsöffnungen 50 und gelangt durch einen Zwischenraum 54, der sich im geöffneten Zustand des zweiten Verschlusselements 26 zwischen diesem und dem ersten Verschlusselement 24 bildet, hindurch bis in einen Auslass 56 des zweiten Verschlusselements 26 (siehe wiederum Fign. 2 und 3).

Wie insbesondere anhand von Fig. 2 zu erkennen ist, weist das zweite Verschlusselement 26 einen Dichtring 58 auf, der sich innerhalb der Einsetzöffnung 36 der Aufnahmekammer 32 befindet, wenn das Behältnis 10 in die Aufnahmekammer 32 eingesetzt ist. Dieser Dichtring 58 dichtet den sich zwischen dem ersten Verschlusselement 24 und der Aufnahmekammer 32 bildenden Ringraum 60 zur Einsetzöffnung 36 und damit nach außen hin ab. Gas, das eventuell durch den Gewindeeingriff 62 zwischen den beiden Verschlusselementen 24, 26 hindurch strömen könnte, wird über einen weiteren Dichtring 64 zwischen dem zweiten Verschlusselement 26 und dem ersten Verschlusselement 24 daran gehindert, nach außen auszutreten. Das zweite Verschlusselement 26 weist ein in diesem Ausführungsbeispiel als radial nach außen ragender Flansch ausgebildetes Anlageelement 66 auf, mit dem es am Rand 68 der Aufnahmekammer 32 aufliegt.

In der zuvor beschriebenen Gebrauchsposition des Behältnisses 10 zum Begasen seines Gefäßes 14 ist also das erste Verschlusselement 24 dicht mit dem Gefäß 14 verbunden und verschließt dessen Befüllöffnung 20 (siehe auch die Dichtung 67), während sich das zweite Verschlusselement 26 in seiner Öffnungsposition befindet, in der sowohl die Gaszuführkanal-Einlässe 40 als auch die Entlüftungsöffnungen 50 freigelegt und damit geöffnet sind. Aus den Entlüftungsöffnungen 50 des ersten Verschlusselements 24 oder aus Öffnung 56 des zweiten Verschlusselements 26 austretendes Gas kann bei Bedarf durch ein (nicht dargestelltes) Neutralisationsmaterial (z. B. als Filter ausgebildet), an dem bzw. durch das das Gas entlang bzw. hindurch strömt, neutralisiert werden. Dieses Neutralisationsmaterial kann außen am Verschlusselement 26 angeordnet und mit einem Halteelement (z. B. auf das Verschlusselement 26 aufgesteckte Kappe oder dergleichen) fixiert sein, wobei durch das Halteelement hindurch oder aber zwischen dem Halteelement und dem zweiten Verschlusselement 26 (und, sofern das Halteelement auch an dem ersten Verschlusselement 24 angreift, auch zwischen dem Halteelement und dem ersten Verschlusselement) für eine Entlüftung zu sorgen ist, was allerdings auch durch einen Filter, der das Neutralisationsmaterial aufweist, erfolgen kann.

Nach dem Begasungsvorgang wird das Behältnis 10 aus der Aufnahmekammer 32 entnommen und das zweite Verschlusselement 26 durch Verschraubung mit dem ersten Verschlusselement 24 in seine Verschlussposition überführt, in der sowohl die Einlässe 40 als auch die Entlüftungsöffnungen 50 verschlossen sind. Dabei wirkt unterstützend eine flache Ringdichtung 70 mit, die auf einer umlaufenden Schulter 72 am ersten Verschlusselement 24 ausgebildet ist. Diese Schulter 72 bildet eine Flanke einer umlaufenden Ringnut 74.

Die Entlüftungsöffnungen 50 werden durch eine Dichtungsscheibe 76 verschlossen, die an dem zweiten Verschlusselement 26 angeordnet ist, und zwar den Entlüftungsöffnungen 50 gegenüberliegend. Diese Situation ist in den Fign. 6 und 7 gezeigt.

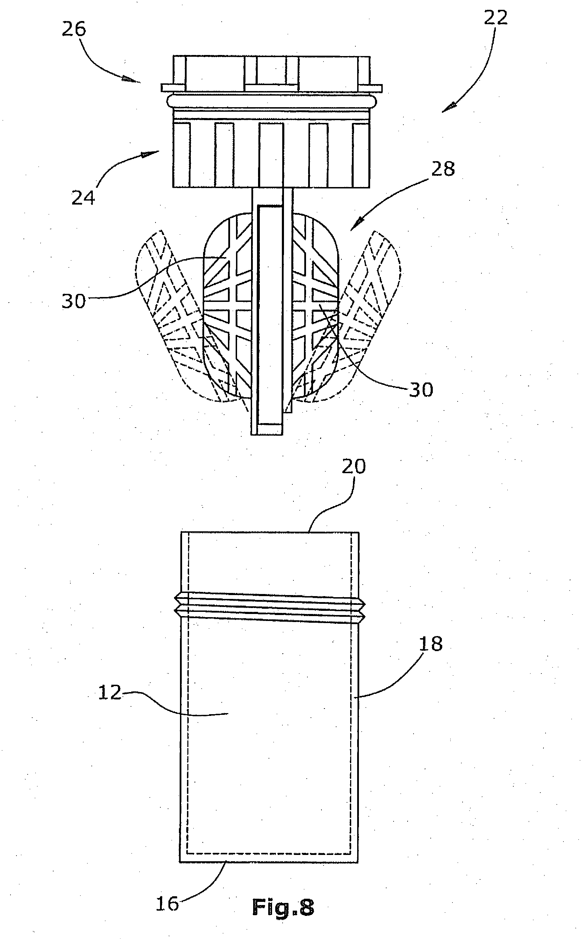

Um nun die Kontaktlinsen bzw. zunächst den Kontaktlinsenhalter 28 aus dem Gefäß 14 entnehmen zu können, wird das erste Verschlusselement 24 vom Gefäß 14 abgeschraubt, wobei dabei die Relativposition zwischen erstem und zweitem Verschlusselement 24 bzw. 26 beibehalten bleibt, das zweite Verschlusselement 26 also weiterhin in seiner Verschlussposition verbleibt. Diese Situation ist beispielsweise in Fig. 8 gezeigt.

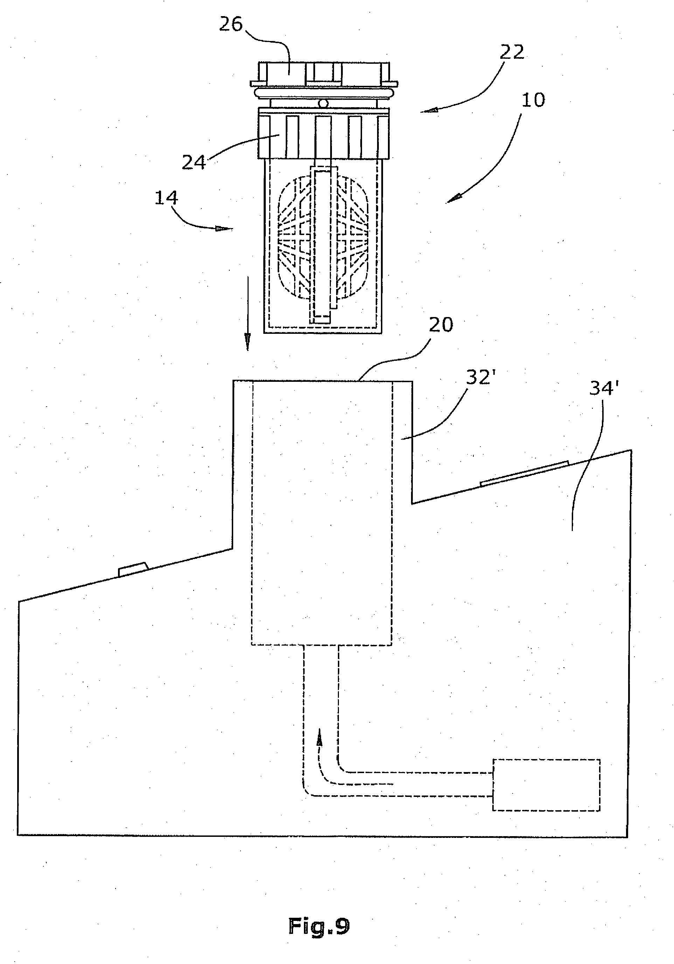

Fig. 9 zeigt ein Ausführungsbeispiel einer Einheit 34 zur Erzeugung des Reinigungs- und/oder Entkeimungsgases, bei der die Aufnahmekammer 32 nicht in die Aufnahme eines Gehäuses der Einheit 34 gemäß Fig. 1, sondern in eine einstückig mit dem Gehäuse der Einheit 34' ausgebildete Aufnahmekammer 32' eingesetzt ist. Das Behältnis 10, das in die Aufnahmekammer 32' einsetzbar ist, ist so, wie anhand der vorherigen Figuren beschrieben, ausgebildet.

Wie weiter oben erwähnt, können die beim Begasen des Innern des Gefäßes 14 aus den Entlüftungsöffnungen 50 austretenden Reinigungs- und/oder Entkeimungsgase (z.B. Ozon) durch ein (Neutralisations-)Filter (z.B. Aktivkohlefilter) neutralisiert werden. Mit der insbesondere automatisch endenden Begasung durch die Einheit 34 zur Erzeugung dieser Gase ist allerdings über die Entlüftungsöffnungen 50 (und das Filter) eine Fluidverbindung zwischen der Umgebung und dem Innern des Gefäßes 14 gegeben. In dieser Phase könnten also Dekontaminationen im Innern des Gefäßes und damit der Kontaktlinse sowie der Aufbewahrungsflüssigkeit entstehen. Um dies zu unterbinden, bietet es sich an, im Bereich der Entlüftungsöffnungen 50 (also vor oder hinter diesen) oder vor oder hinter dem gegebenenfalls vorhandenen (Neutralisations-)Filter ein Einweg- oder Rückschlag-Ventil vorzusehen, das infolge des Drucks der austretenden Gase automatisch öffnet und dann, wenn dieser Druck nicht mehr ansteht, automatisch schließt. Ein solches Ventil kann einen Ventilkörper aufweisen, der infolge einer Vorspannung oder unter dem Einfluss seines Eigengewichts dichtend an einem Ventilsitz des Ventils anliegt. Der Ventilkörper wird dann bei einer anstehenden Gasströmung automatisch angehoben bzw. allgemein ausgedrückt vom Ventilsitz wegbewegt. Der Ventilkörper kann grundsätzlich beliebig ausgestaltet und z.B. als bewegbarer Kolben oder Konus oder als bewegbare Kugel ausgebildet sein. Alternativ kann das Ventil auch einen scheiben- oder klappenförmigen gegebenenfalls flexiblen Ventilkörper aufweisen, der die Entlüftungsöffnungen 50 in der Schließstellung überdeckt und z.B. sich infolge seiner flexiblen Anbindung von den Entlüftungsöffnungen zumindest in einem Teilbereich zur Entlüftung des Gefäßes beim Begasen webbewegt und diese freigibt.

Es versteht sich von selbst, dass das Ventil wie sämtliche anderen Teile des Gefäßes, die in Kontakt mit dem Reinigungs- und/oder Entkeimungsgas sowie der Aufbewahrungsflüssigkeit stehen, gegenüber diesen inert sind.

## Patentansprüche

1. Behältnis für eine Kontaktlinsen-Aufbewahrungsflüssigkeit, insbesondere mit in dieser befindlichen Kontaktlinsen, mit
- einem Gefäß (14), das eine Befüllöffnung (20) aufweist,
- einem an dem Gefäß (14) anbringbaren ersten Verschlusselement (24) zum Verschließen der Befüllöffnung (20),
- wobei das erste Verschlusselement (24) einen Gaszuführkanal (42) für ein Reinigungs- und/oder Entkeimungsgas aufweist, der sich von mindestens einem Einlass (40) an einer Außenseite des ersten Verschlusselements (24) bis zumindest einem Auslass (48) an einer zum Innern des Gefäßes (14) weisenden Innenseite des ersten Verschlusselements (24) erstreckt, und
- wobei das erste Verschlusselement (24) an seiner Außenseite mindestens eine Entlüftungsöffnung (50) für den Austritt von Gas aus dem Gefäß (14) aufweist, und
- einem an dem ersten Verschlusselement (24) angeordneten zweiten Verschlusselement (26) zum Verschließen des mindestens einen Einlasses (40) des Gaszuführkanals (42) und der mindestens einen Entlüftungsöffnung (50) des ersten Verschlusselements (24),
- wobei an dem ersten Verschlusselement (24) ein Kontaktlinsenhalter (28) angeordnet ist, durch den sich ein Gasweiterleitungskanal (46) erstreckt, welcher in Fluidverbindung mit dem Gaszuführkanal (42) des ersten Verschlusselements (24) steht.

2. Behältnis nach Anspruch 1, **dadurch gekennzeichnet, dass** die Außenseite des ersten Verschlusselements (24) in dessen an dem Gefäß (14) angebrachten Zustand einen von dem Gefäß (14) aufragenden ersten Außenflächenbereich (44) aufweist, innerhalb dessen der mindestens eine Einlass (40) angeordnet ist.

3. Behältnis nach Anspruch 1 oder 2, **dadurch gekennzeichnet, dass** die Außenseite des ersten Verschlusselements (24) in dessen an dem Gefäß (14) angebrachten Zustand einen im Wesentlichen parallel zur Erstreckung der Befüllöffnung (20) verlaufenden zweiten Außenflächenbereich (52) aufweist, innerhalb dessen die Entlüftungsöffnung (50) des ersten Verschlusselements (24) angeordnet ist.

4. Behältnis nach Anspruch 2 oder 3, **dadurch gekennzeichnet, dass** das zweite Verschlusselement (26) in einer Verschlussposition sowohl den mindestens einen Gaszuführkanal-Einlass (40) als auch die Entlüftungsöffnung (50) des ersten Verschlusselements (24) überdeckt.

5. Behältnis nach einem der Ansprüche 1 bis 4, **dadurch gekennzeichnet, dass** das Gefäß (14) eine Bodenwand (16) mit einer von dieser aufragenden umlaufenden Mantelwand (18), insbesondere Zylindermantelwand, aufweist und dass die Befüllöffnung (20) des Gefäßes (14) der Bodenwand (16) gegenüberliegend angeordnet ist.

6. Behältnis nach Anspruch 5, **dadurch gekennzeichnet, dass** die Mantelwand (18) des Gefäßes (14) einen dessen Bodenwand (16) gegenüberliegenden umlaufenden Rand aufweist, der die Befüllöffnung (20) begrenzt.

7. Behältnis nach einem der Ansprüche 1 bis 6, **dadurch gekennzeichnet, dass** das erste Verschlusselement (24) auf das Gefäß (14) aufsteck- oder aufsetzbar ist oder in die Befüllöffnung (20) einsteckbar oder mit dem Gefäß (14) verschraubbar oder an dem Gefäß (14) durch Verdrehen, beispielsweise mittels einer Bajonett-Verbindung, anbringbar ist und/oder dass das zweite Verschlusselement (26) auf das erste Verschlusselement (24) aufsteck- oder aufsetzbar oder mit dem ersten Verschlusselement (24) verschraubbar oder an dem ersten Verschlusselement (24) durch Verdrehen, beispielsweise mittels einer Bajonett-Verbindung, anbringbar ist.

8. Behältnis nach Anspruch 2 oder nach 3 bis 7, soweit auf Anspruch 2 rückbezogen, **dadurch gekennzeichnet, dass** der Einlass (40) des Gaszuführkanals (42) des ersten Verschlusselements (24) als zumindest teilweise um das Verschlusselement umlaufende Ringnut (74) in dem ersten Außenflächenbereich (44) ausgebildet ist und dass das erste Verschlusselement (24) ein Dichtelement (70) aufweist, an dem das zweite Verschlusselement (26) beim Überdecken der Ringnut (74) dichtend anliegt.

9. Behältnis nach einem der Ansprüche 1 bis 8, **dadurch gekennzeichnet, dass** das zweite Verschlusselement (26) eine Auslassöffnung (56) aufweist, die seitlich versetzt zur Entlüftungsöffnung (50) des ersten Verschlusselements (24) angeordnet ist, und dass um die Entlüftungsöffnung (50) des ersten Verschlusselements (24) und/oder um die Auslassöffnung (56) des zweiten Verschlusselements (26) herum oder an dem zweiten Verschlusselement (26) der Entlüftungsöffnung (50) gegenüberliegend und/oder an dem ersten Verschlusselement (24) der Auslassöffnung (56) gegenüberliegend ein Dichtungselement (76) angeordnet ist, an dem das zweite bzw. das erste Verschlusselement (24,26) in der Verschlussposition des zweiten Verschlusselements (26) dichtend anliegt.

10. Behältnis nach einem der Ansprüche 1 bis 9, **dadurch gekennzeichnet, dass** beide Verschlusselemente (24,26) jeweils als erste bzw. zweite Schraubverschlussdeckel ausgebildet sind, wobei der erste Schraubverschlussdeckel mit dem Gefäß (14) und der zweite Schraubverschlussdeckel mit dem ersten Schraubverschlussdeckel verschraubbar ist.

11. Behältnis nach Anspruch 10, **dadurch gekennzeichnet, dass** die Schraubverschlussdeckel als erste bzw. zweite Schraubkappen ausgebildet sind, die jeweils einen Umfangsabschnitt (44) und einen Stirnseitenabschnitt (52) an ihren Außenseiten aufweisen.

12. Behältnis nach Anspruch 11, **dadurch gekennzeichnet, dass** der Einlass (40) des Gaszuführkanals der ersten Schraubkappe an deren Umfangsabschnitt (44) ausgebildet ist und der Umfangsabschnitt (44) der zweiten Schraubkappe den Einlass (40) des Gaszuführkanals (42) der ersten Schraubkappe zum Verschließen des Einlasses (40) überdeckt und dass die Entlüftungsöffnung (50) der ersten Schraubkappe in deren Stirnseitenabschnitt (52) ausgebildet ist und der Stirnseitenabschnitt der zweiten Schraubkappe die Entlüftungsöffnung (50) der ersten Schraubkappe zum Verschließen der Entlüftungsöffnung (50) insbesondere mittels eines Dichtungselements (76) überdeckt.

13. Behältnis nach einem der Ansprüche 1 bis 12, **dadurch gekennzeichnet, dass** jedes Verschlusselement (24,26) manuell aus einer Öffnungsposition in eine Verschlussposition und umgekehrt überführbar ist, wobei beide Verschlusselemente (24,26) in der jeweiligen Verschlussposition an dem Gefäß (14) bzw. dem ersten Verschlusselement (24) verbleiben und ein mechanischer Widerstand gegen eine aus der betreffenden Verschlussposition kommende Überführung über die betreffende Öffnungsposition hinaus oder umgekehrt gegeben ist.

14. Behältnis nach einem der Ansprüche 1 bis 13, **gekennzeichnet durch** eine eine Einsetzöffnung (36) aufweisende Aufnahmekammer (32) zur Aufnahme des Gefäßes (14), wobei das zweite Verschlusselement (26) ein Anlageelement (66) zur Anlage an der Einsetzöffnung (36) der Aufnahmekammer (32) bei von dieser unter Bildung eines Ringraums (60) zwischen dem Einlass (40) des Gaszuführkanals (42) des ersten Verschlusselements (24) und der Aufnahmekammer (32) aufgenommenen Gefäß (14) aufweist, und dass zwischen der Einsetzöffnung (36) und dem Einlass (40) des Gaszuführkanals (42) des ersten Verschlusselements (24) ein den Ringraum (60) zur Einsetzöffnung (36) hin abdichtendes Dichtungselement (58) angeordnet ist.

15. Behältnis nach Anspruch 14, **dadurch gekennzeichnet, dass** das Dichtungselement (58) in der Einsetzöffnung (36) der Aufnahmekammer (32) oder um die Einsetzöffnung (36) der Aufnahmekammer (32) herum zur Anlage an dem Anlageelement (66) des zweiten Verschlusselements (26) oder an dem ersten Verschlusselement (24) zwischen dem mindestens einen Einlass (40) des Gaszuführkanals (42) und dem Anlageelement (66) des zweiten Verschlusselements (26) angeordnet ist.

16. Behältnis nach einem der Ansprüche 1 bis 15, **dadurch gekennzeichnet, dass** das zweite Verschlusselement (26) ein Neutralisationsmaterial zum Neutralisieren von aus der Entlüftungsöffnung (50) des ersten Verschlusselements (24) entweichendes Gas vor dessen Entweichen in die Umgebung aufweist.

17. Reinigungs- und/oder Entkeimungsvorrichtung für in einer Kontaktlinsenaufbewahrungsflüssigkeit befindliche Kontaktlinsen mit
- einem Behältnis (10) nach einem der Ansprüche 1 bis 13 und
- einer ein Reinigungs- und/oder Entkeimungsgas erzeugende und/oder freigebende Einheit (34,34') mit einer Aufnahmekammer (32,32') nach Anspruch 14 oder 15.

18. Reinigungs- und/oder Entkeimungsvorrichtung nach Anspruch 17, wobei die Aufnahmekammer (32') einstückig mit der Reinigungs- und/oder Entkeimungsgas erzeugende und/oder freigebende Einheit (34') ausgebildet ist oder ein von dieser Einheit (34) getrenntes Teil ist, das in eine Aufnahme in der Einheit (34) einsetzbar ist.

## Claims

1. A container for a contact lens storage solution, in particular having contact lenses in said contact lens storage solution, said container comprising
- a vessel (14) having a filling opening (20),
- a first closing element (24) attachable to the vessel (14) for closing the filling opening (20),
- the first closing element (24) comprising a gas supply channel (42) for a cleaning and/or sterilizing gas, which extends from at least one inlet (40) on an outer face of the first closing element (24) to at least one outlet (48) on an inner face of the first closing element (24) facing the interior of the vessel (14), and
- said first closing element (24) comprising, on its outer face, at least one venting opening (50) for the escape of gas from the vessel (14), and
- a second closing element (26) arranged on the first closing element (24), for closing the at least one inlet (40) of the gas supply channel (42) and the at least one venting opening (50) of the first closing element (24,
- the first closing element (24) having arranged on it a contact lens holder (28) with a gas forwarding channel (46) extending therethrough which is in fluid connection with the gas supply channel (42) of the first closing element (24).

2. The container according to claim 1, **characterized in that** the outer face of the first closing element (24), in the state where said element is mounted to the vessel (14), comprises a first outer face area (44) extending upward from the vessel (14) and having arranged in it the at least one inlet (40).

3. The container according to claim 1 or 2, **characterized in that** the outer face of the first closure element (24), in the state where said element is mounted to the vessel (14), comprises a second outer face area (52) arranged substantially parallel to the extension of the filling opening (20) and having arranged in it the venting opening (50) of the first closure element (24).

4. The container according to claim 2 or 3, **characterized in that** the second closure element (26) in a closing position covers both said at least one gas supply channel inlet (40) and the venting opening (50) of the first closure element (24).

5. The container according to any one of claims 1 to 4, **characterized in that** the vessel (14) comprises a bottom wall (16) having a circumferential enclosure wall (18), particularly a cylindrical enclosure wall, extending upward from it, and that the filling opening (20) of the vessel (14) is arranged opposite to said bottom wall (16).

6. The container according to claim 5, **characterized in that** the enclosure wall (18) of the vessel (14) comprises a circumferential edge arranged opposite to the bottom wall (16) of the vessel and delimiting the filling opening (20).

7. The container according to any one of claims 1 to 6, **characterized in that** the first closure element (24) can be plugged or mounted onto the vessel (14), or can be inserted into the filling opening (20), or can be fastened to the vessel (14) by threaded engagement or can be fastened to the vessel (14) by twisting, e.g. by means of a bayonet lock, and/or that the second closure element (26) can be plugged or mounted to the first closure element (24), or can be fastened to the first closure element (24) by threaded engagement or can be fastened to the first closure element (24) by twisting, e.g. by means of a bayonet lock.

8. The container according to claim 2 or according to any one of claims 3 to 7 as far as dependent on claim 2, **characterized in that** the inlet (40) of the gas supply channel (42) of the first closure element (24) is formed in the first outer face area (44) as an annular groove (74) at least partially surrounding the closure element, and that the first closure element (24) comprises a sealing element (70) on which the second closure element (26) is in sealed abutment when covering said annular groove (74).

9. The container according to any one of claims 1 to 8, **characterized in that** the second closure element (26) comprises an outlet opening (56) arranged at a lateral displacement relative to the venting opening (50) of the first closure element (24), and that, around the venting opening (50) of the first closure element (24), and/or around the outlet opening (56) of the second closure element (26), or on the second closure element (26) opposite to the venting opening (50), and/or on the first closure element (24) opposite to the outlet opening (56), a sealing element (76) is arranged which is sealingly abutted by the second and respectively the first closure element (24,26) in the closed position of the second closure element (26).

10. The container according to any one of claims 1 to 9, **characterized in that** both closure elements (24,26) are formed as first and respectively second screw closure lids, the first screw closure lid being adapted for threaded engagement with the vessel (14) and the second screw closure lid being adapted for threaded engagement with the first screw closure lid.

11. The container according to claim 10, **characterized in that** said screw closure lids are formed as first and respectively second screw caps which each comprise a peripheral portion (44) and an end side portion (52) on their outer faces.

12. The container according to claim 11, **characterized in that** the inlet (40) of the gas supply channel of the first screw cap is formed at the peripheral portion (44) of the first screw cap, and the peripheral portion (44) of the second screw cap covers the inlet (40) of the gas supply channel (42) of the first screw cap for closing the inlet (40), and that the venting opening (50) of the first screw cap is formed in the end face portion (52) of the first screw cap and the end face portion of the second screw cap covers the venting opening (50) of the first screw cap for closing the venting opening (50), particularly by means of a sealing element (76).

13. The container according to any one of claims 1 to 12, **characterized in that** each closure element (24,26) can be manually transferred from an opening position into a closing position or vice versa, wherein both closure elements (24,26) remain in the respective closing position on the vessel (14) and respectively the first closure element (24), and a mechanical resistance exists against a transfer from out of the respective closing position beyond the respective opening position and vice versa.

14. The container according to any one of claims 1 to 13, **characterized by** a receiving chamber (32) comprising an insertion opening (36) and provided for receiving the vessel (14), wherein the second closure element (26) comprises an abutment element (66) for abutment on the insertion opening (36) of the receiving chamber (32) in the state where the receiving chamber receives the vessel (14) while forming an annular space (60) between the inlet (40) of the gas supply channel (42) of the first closure element (24) and the receiving chamber (32), and that, between the insertion opening (36) and the inlet (40) of the gas supply channel (42) of the first closure element (24), a sealing element (58) is arranged for sealing said annular space (60) toward the insertion opening (36).

15. The container according to claim 14, **characterized in that** the sealing element (58) is arranged in the insertion opening (36) of the receiving chamber (32) or around the insertion opening (36) of the receiving chamber (32) so as to be in abutment on said abutment element (66) of the second closure element (26) or on the first closure element (24), the latter abutment between the at least one inlet (40) of the gas supply channel (42) and the abutment element (66) of the second closure element (26).

16. The container according to any one of claims 1 to 15, **characterized in that** the second closure element (26) comprises a neutralization material for neutralizing gas escaping from the venting opening (50) of the first closure element (24) prior to escape of said gas into the ambience.

17. A cleaning and/or sterilizing device for contact lenses arranged in a contact lens storage solution, said device comprising
- a container (10) according to any one of claims 1 to 13, and
- a unit (34,34') provided for generating and/or releasing a cleaning and/or sterilizing gas, said unit comprising a receiving chamber (32,32') according to claim 14 or 15.

18. The cleaning and/or sterilizing device according to claim 17, wherein said receiving chamber (32') is formed integrally with said unit (34') provided for generating and/or releasing a cleaning and/or sterilizing gas, or is a component which is separated from said unit (34) and insertable into a receiving portion in said unit (34).

## Revendications

1. Récipient pour un liquide de stockage de lentilles de contact, en particulier avec des lentilles de contact situées dans ce liquide, avec
- un réservoir (14) qui présente une ouverture de remplissage (20),
- un premier élément de fermeture (24) pouvant être mis en place sur le réservoir (14) pour la fermeture de l'ouverture de remplissage (20),
- le premier élément de fermeture (24) présentant un canal d'amenée de gaz (42) pour un gaz de nettoyage et/ou de désinfection qui s'étend à partir d'au moins une entrée (40) sur un côté extérieur du premier élément de fermeture (24) jusqu'à au moins une sortie (48) sur un côté intérieur, tourné vers l'intérieur du réservoir (14), du premier élément de fermeture (24), et
- le premier élément de fermeture (24) présentant, sur son côté extérieur, au moins une ouverture d'évent (50) pour la sortie du gaz hors du réservoir (14), et
- un deuxième élément de fermeture (26), disposé sur le premier élément de fermeture (24), pour la fermeture de l'entrée (40) au moins au nombre de un du canal d'amenée de gaz (42) et de l'ouverture d'évent (50) au moins au nombre de un du premier élément de fermeture (24),
- un élément de retenue de lentilles de contact (28), à travers lequel s'étend un canal de transport du gaz (46), étant disposé sur le premier élément de fermeture (24), et le canal de transport du gaz (46) étant en liaison fluidique avec le canal d'amenée de gaz (42) du premier élément de fermeture (24).

2. Récipient selon la revendication 1, **caractérisé en ce que** le côté extérieur du premier élément de fermeture (24), dans son état mis en place sur le réservoir (14), présente une première zone de surface extérieure (44), dépassant du réservoir (14), à l'intérieur de laquelle est disposée l'entrée (40) au moins au nombre de un.

3. Récipient selon la revendication 1 ou 2, **caractérisé en ce que** le côté extérieur du premier élément de fermeture (24), dans son état mis en place sur le réservoir (14), présente une deuxième zone de surface extérieure (52) agencée essentiellement parallèlement à l'étendue de l'ouverture de remplissage (20) et à l'intérieur de laquelle zone est disposée l'ouverture d'évent (50) du premier élément de fermeture (24).

4. Récipient selon la revendication 2 ou 3, **caractérisé en ce que** le deuxième élément de fermeture (26), dans une position de fermeture, recouvre aussi bien l'entrée (40) de canal d'amenée de gaz au moins au nombre de un que l'ouverture d'évent (50) du premier élément de fermeture (24).

5. Récipient selon une des revendications 1 à 4, **caractérisé en ce que** le réservoir (14) présente une paroi de fond (16) avec une paroi d'enveloppe (18) périphérique dépassant de la paroi de fond, en particulier une paroi d'enveloppe cylindrique, et **en ce que** l'ouverture de remplissage (20) du réservoir (14) est disposée en face de la paroi de fond (16).

6. Récipient selon la revendication 5, **caractérisé en ce que** la paroi d'enveloppe (18) du réservoir (14) présente un bord périphérique qui est situé en face de la paroi de fond (16) du réservoir et qui limite l'ouverture de remplissage (20).

7. Récipient selon une des revendications 1 à 6, **caractérisé en ce que** le premier élément de fermeture (24) peut être emmanché ou rapporté sur le réservoir (14) ou peut être enfiché dans l'ouverture de remplissage (20) ou peut être assemblé par vissage au réservoir (14) ou peut être mis en place sur le réservoir (14) par torsion, par exemple au moyen d'une liaison à baïonnette, et/ou en ce que le deuxième élément de fermeture (26) peut être emmanché ou rapporté sur le premier élément de fermeture (24) ou peut être assemblé par vissage au premier élément de fermeture (24) ou peut être mis en place sur le premier élément de fermeture (24) par torsion, par exemple au moyen d'une liaison à baïonnette.

8. Récipient selon la revendication 2 ou selon les revendications 3 à 7 dans la mesure où elles font référence à la revendication 2, **caractérisé en ce que** l'entrée (40) du canal d'amenée de gaz (42) du premier élément de fermeture (24) est conformée, dans la zone de surface extérieure (44), en rainure (74) annulaire entourant au moins partiellement l'élément de fermeture et **en ce que** le premier élément de fermeture (24) présente un élément d'étanchéité (70) sur lequel le deuxième élément de fermeture (26) s'appuie de façon étanche lors du recouvrement de la rainure (74) annulaire.

9. Récipient selon une des revendications 1 à 8, **caractérisé en ce que** le deuxième élément de fermeture (26) présente une ouverture de sortie (56) qui est disposée avec un décalage latéral par rapport à l'ouverture d'évent (50) du premier élément de fermeture (24), et **en ce qu'**un élément d'étanchéité (76) est disposé autour de l'ouverture d'évent (50) du premier élément de fermeture (24) et/ou autour de l'ouverture de sortie (56) du deuxième élément de fermeture (26) ou en face de l'ouverture d'évent (50) sur le deuxième élément de fermeture (26) et/ou en face de l'ouverture de sortie (56) sur le premier élément de fermeture (24), élément d'étanchéité sur lequel appuie de façon étanche le deuxième ou respectivement le premier élément de fermeture (24, 26) dans la position de fermeture du deuxième élément de fermeture (26).

10. Récipient selon une des revendications 1 à 9, **caractérisé en ce que** les deux éléments de fermeture (24, 26) sont constitués respectivement en tant que premier ou respectivement deuxième couvercle de fermeture à vis, le premier couvercle de fermeture à vis pouvant être assemblé par vissage au réservoir (14), et le deuxième couvercle de fermeture à vis pouvant être assemblé par vissage avec le premier couvercle de fermeture à vis.

11. Récipient selon la revendication 10, **caractérisé en ce que** les couvercles de fermeture à vis sont constitués en tant que premier ou respectivement deuxième capuchon à vis, qui présentent respectivement un tronçon périphérique (44) et un tronçon frontal (52) sur leurs côtés extérieurs.

12. Récipient selon la revendication 11, **caractérisé en ce que** l'entrée (40) du canal d'amenée de gaz du premier capuchon à vis est constituée sur le tronçon périphérique (44) de celui-ci, et **en ce que** le tronçon périphérique (44) du deuxième capuchon à vis recouvre l'entrée (40) du canal d'amenée de gaz (42) du premier capuchon à vis pour la fermeture de l'entrée (40), et **en ce que** l'ouverture d'évent (50) du premier capuchon à vis est constituée dans le tronçon frontal (52) de celui-ci, et le tronçon frontal du deuxième capuchon à vis recouvre l'ouverture d'évent (50) du premier capuchon à vis pour la fermeture de l'ouverture d'évent (50), en particulier au moyen d'un élément d'étanchéité (76).

13. Récipient selon une des revendications 1 à 12, **caractérisé en ce que** chaque élément de fermeture (24, 26) peut être transféré manuellement à partir d'une position d'ouverture vers une position de fermeture et inversement, les deux éléments de fermeture (24, 26) demeurant dans la position de fermeture respective sur le réservoir (14) respectivement sur le premier élément de fermeture (24), et une résistance mécanique, s'opposant à un transfert partant de la position de fermeture concernée et allant au-delà de la position d'ouverture concernée ou inversement, étant réalisée.

14. Récipient selon une des revendications 1 à 13, **caractérisé par** une chambre de réception (32) présentant une ouverture d'introduction (36), pour la réception du réservoir (14), le deuxième élément de fermeture (26) présentant un élément d'appui (66) pour l'appui sur l'ouverture d'introduction (36) de la chambre de réception (32), le réservoir (14) étant reçu par celle-ci, avec formation d'un espace annulaire (60) entre l'entrée (40) du canal d'amenée de gaz (42) du premier élément de fermeture (24) et la chambre de réception (32), et en ce que, entre l'ouverture d'introduction (36) et l'entrée (40) du canal d'amenée de gaz (42) du premier élément de fermeture (24), il est disposé un élément d'étanchéité (58) assurant l'étanchéité de l'espace annulaire (60) vis-à-vis de l'ouverture d'introduction (36).

15. Récipient selon la revendication 14, **caractérisé en ce que** l'élément d'étanchéité (58) est disposé dans l'ouverture d'introduction (36) de la chambre de réception (32) ou autour de l'ouverture d'introduction (36) de la chambre de réception (32) pour l'appui sur l'élément d'appui (66) du deuxième élément de fermeture (26) ou sur le premier élément de fermeture (24) entre l'entrée (40), au moins au nombre de un, du canal d'amenée de gaz (42) et l'élément d'appui (66) du deuxième élément de fermeture (26).

16. Récipient selon une des revendications 1 à 15, **caractérisé en ce que** le deuxième élément de fermeture (26) présente un matériau de neutralisation pour la neutralisation du gaz s'échappant de l'ouverture d'évent (50) du premier élément de fermeture (24) avant qu'il n'atteigne l'environnement.

17. Dispositif de nettoyage et/ou de désinfection pour des lentilles de contact situées dans un liquide de stockage de lentilles de contact, avec
- un récipient (10) selon une des revendications 1 à 13, et
- une unité (34, 34') produisant et/ou dégageant un gaz de nettoyage et/ou de désinfection, avec une chambre de réception (32, 32') selon la revendication 14 ou 15.

18. Dispositif de nettoyage et/ou de désinfection selon la revendication 17, la chambre de réception (32') étant constituée d'un seul tenant avec l'unité (34') produisant et/ou dégageant un gaz de nettoyage et/ou de désinfection, ou bien étant une partie qui est séparée de cette unité (34) et qui peut être introduite dans un logement dans l'unité (34).
